# EUROPÄISCHE PATENTSCHRIFT

(11) **EP 2 798 343 B1**
(45) Veröffentlichungstag und Bekanntmachung des Hinweises auf die Patenterteilung: **30.09.2020**
(21) Anmeldenummer: 12816669.1
(22) Anmeldetag: 27.12.2012
(51) Int. Cl.: G01N 33/22, G01N 27/62

(54) **VERFAHREN ZUR BESTIMMUNG DES GEHALTES AN ORGANISCHEN SILIZIUMVERBINDUNGEN IN ANTHROPOGENEN UND/ODER BIOGENEN, METHANHALTIGEN GASEN**
METHOD FOR DETERMINING THE CONTENT OF ORGANIC SILICON COMPOUNDS IN ANTHROPOGENIC AND/OR BIOGENIC GASES CONTAINING METHANE
PROCÉDÉ DE DÉTERMINATION DE LA TENEUR EN COMPOSÉS DE SILICIUM ORGANIQUES DE GAZ ANTHROPOGÈNES ET/OU BIOGÈNES CONTENANT DU MÉTHANE

(30) Priorität: 28.12.2011 DE 102011057096; 08.03.2012 DE 102012101945
(43) Veröffentlichungstag der Anmeldung: 05.11.2014
(73) Patentinhaber: IMSPEX DIAGNOSTICS Ltd, Rhondda Cynon Taff CF45 4SN (GB)
(72) Erfinder: WORTELMANN, Thomas, 44141 Dortmund (DE); SIELEMANN, Stefanie, 44143 Dortmund (DE)
(74) Vertreter: Meinke, Jochen
(86) Internationale Anmeldenummer: PCT/EP2012/076916
(87) Internationale Veröffentlichungsnummer: WO 2013/098299

(56) Entgegenhaltungen:
- FR-A1- 2 886 409
- JP-A- 2008 196 870
- ALEXANDER BUNKOWSKI ET AL: "One-year time series of investigations of analytes within human breath using ion mobility spectrometry", INTERNATIONAL JOURNAL FOR ION MOBILITY SPECTROMETRY, Bd. 13, Nr. 3-4, 7. November 2010 (2010-11-07), Seiten 141-148, XP055061712, ISSN: 1435-6163, DOI: 10.1007/s12127-010-0052-7
- ARA B SAHAKIAN ET AL: "Methane and the Gastrointestinal Tract", DIGESTIVE DISEASES AND SCIENCES, KLUWER ACADEMIC PUBLISHERS-PLENUM PUBLISHERS, NE, vol. 55, no. 8, 15 October 2009 (2009-10-15), pages 2135-2143, XP019816463, ISSN: 1573-2568

## Beschreibung

Die Erfindung betrifft ein Verfahren zur Bestimmung des Gehaltes an organischen Siliziumverbindungen in anthropogenen und/oder biogenen, methanhaltigen Gasen, wie Klärgasen, Deponiegasen.

Bei der energetischen Nutzung biogener oder anthropogener metallhaltiger Gase, wie Biogas, Klärgas oder Deponiegas, treten in der Praxis verschiedene Probleme auf, welche insbesondere durch die in den Gasen enthaltenen organischen Siliziumverbindungen (Siloxane) hervorgerufen werden.

Organische Anteile von z.B. in einer Deponie abgelagerten Haus- und Gewerbeabfällen werden im Laufe der Zeit biologisch durch Mikroorganismen abgebaut. Am Ende dieser biologischen Umsetzungskette stehen Methanbakterien, die die restorganischen Bestandteile unter Sauerstoffausschluss (anaerob) zu Methan und Kohlendioxid umsetzen. Dieses Gasgemisch, das in der Regel zwischen 50 und 60 % Methan und 35 bis 45 % Kohlendioxid enthält, wird als Deponiegas bezeichnet. Methan verstärkt den Treibhauseffekt und ist somit klimaschädlich, wenn es unkontrolliert in die Umwelt entweicht. Zudem ist es aufgrund seiner leichten Entflammbarkeit unter bestimmten Bedingungen explosiv.

Deshalb wird das Deponiegas mittels eines Gasfassungssystems gezielt aus dem Abfallkörper abgesaugt und einer energetischen Verwertung in Gasmotoren zugeführt. Dies ist ökologisch und ökonomisch sinnvoll, da hierdurch das klimaschädliche Methan aus dem Abfall klimaneutral zu Energie (Strom) umgewandelt wird.

Ein Gasfassungssystem besteht in der Regel im Wesentlichen aus einer Vielzahl von vertikalen Gasbrunnen, die in einem Raster von z.B. 50 m im Abfallkörper eingebaut sind. Mit Hilfe dieser Gasbrunnen wird das entstehende Deponiegas durch Unterdruck, der in Verdichtern erzeugt wird, über Gasleitungen Gasmotoren zur elektrischen Energieerzeugung zugeführt. Bevor das Gas die Motoren erreicht, wird das Deponiegas gezielt an mehreren Stellen entwässert.

In der Regel sind vor den Gasmotoren im Gassystem Filter installiert, mit denen das Deponiegas gereinigt wird, meist zunächst mit einem Hauptfilter und einem zweiten nachgeschalteten Filter, der auch als Polizeifilter bezeichnet wird und ein Durchbrechen der Substanzen bei Belegung des Hauptfilters verhindern soll.

Bei diesen Filtern handelt es sich in der Regel um Aktivkohlefilter, die dazu geeignet sind, siliziumorganische Verunreinigungen aus dem Gas herauszufiltern. Solche Siloxane entstehen bei der Verstoffwechselung durch die in Deponien und Klärwerken vorhandenen Bakterien von z.B. Waschmitteln, Kosmetika, anderen Hautpflegeprodukten, silikonbasierten Ölen, Imprägniermitteln, wasserresistenten Materialien, Schuhcremes, Oberflächenschutzmittel, usw. Bei hohen Konzentrationen dieser Siloxane kommt es zu einer Beschädigung der Gasmotoren, da sich die Siloxane auf den Kolben und Ventilen der Gasmotoren ablagern. Beim Absplittern dieser Verkrustungen werden die Ventile undicht und brennen durch, so dass der Zylinderkopf gewechselt werden muss. Dies führt zu reduzierter Standzeit der Anlage und damit zu wirtschaftlichem Schaden. Außerdem erhöhen sich die Wartungskosten und - zeiten der Gasmotoren. Zudem führen Ablagerungen in den Katalysatoren und Thermoreaktionen dazu, dass diese nicht mehr effektiv arbeiten.

Um diese schadhaften Einflüsse auf die Gasmotoren zu vermeiden, dienen die vorerwähnten Filter, deren Funktionsfähigkeit jedoch gewährleistet sein muss. Bisher ist es dazu in der Praxis üblich, von Zeit zu Zeit eine Gasprobe zu entnehmen und diese in einem Labor auf den jeweiligen Siloxangehalt hin bestimmen zu lassen, worauf dann festgestellt werden kann, ob ein Filterwechsel erforderlich ist oder nicht. Ein solches Messverfahren ist jedoch kosten- und zeitaufwendig und benötigt in der Regel mehrere Tage. Dies führt in der Praxis dazu, dass Filter zu spät gewechselt werden, so dass die Gasmotoren durch die Siloxane geschädigt werden können, oder dass prophylaktisch Filter zu früh gewechselt werden, was mit unnötigen zusätzlichen Kosten verbunden ist.

Es besteht somit das Bedürfnis, den Siloxangehalt bei derartigen Anlagen kontinuierlich oder quasi kontinuierlich überprüfen zu können, um zur richtigen Zeit einen Filterwechsel vornehmen zu können. Zu diesem Zweck sind bereits Echtzeitmessungen mittels Infrarotsiloxananalyseeinrichtungen bekannt geworden (z.B. JP 2008196870 A oder JP2011033636A). Diese Messverfahren haben sich aber bisher in der Praxis nicht durchgesetzt, da sie nicht ausreichend selektiv sind und von schwankendem Feuchtegehalt des Gases beeinflusst werden.

Aus FR 2 886 409 A1 ist ein Verfahren zur Bestimmung des Gehaltes an organischen Siliziumverbindungen bekannt, bei welchem die Spektrometrie als Messverfahren verwendet wird. Dabei wird aus dem zu analysierenden Gas eine Probe entnommen, diese Probe nachfolgend in einem Lösungsmittel gelöst und anschließend diese somit flüssige Probe ausgewertet, und zwar vorzugsweise durch plasmaangeregte Atomemissionsspektrometrie, also ein Verfahren zur Analyse flüssiger Proben. Das entnommene Gas kann somit nicht mittels Spektroskopie direkt analysiert werden, sondern es ist erforderlich, die entnommene Gasprobe in einem Lösungsmittel vorzugsweise sogar mehrstufig zu lösen und anschließend die somit flüssige Probe zu analysieren. Dieses Verfahren ist somit sehr aufwendig.

Aus der Veröffentlichung ALEXANDER BUNKOWSKI ET AL, "One-year time series of investigations of analytes within human breath using ion mobility spectrometry", INTERNATIONAL JOURNAL FOR ION MOBILITY SPECTROMETRY, (20101107), vol. 13, no. 3-4, doi:10.1007/s12127-010-0052-7, ISSN 1435-6163, Seiten 141 - 148, ist es bekannt, die Ionenmoblitätsspektrometrie zur direkten Bestimmung volatiler Analyten, z.B. auch Decamethylcyclopentasiloxan, in der menschlichen Atemluft zu bestimmen. Es handelt sich aber ausschließlich um einen medizinisches Einsatz der Ionenmobilitätsspektrometrie unter Laborbedingungen.

Aufgabe der Erfindung ist es, eine Lösung zu schaffen, mit der auf möglichst einfache und zuverlässige Weise der Gehalt an organischen Siliziumverbindungen in anthropogenen und/oder biogenen methanhaltigen Gasen in Biogas-, Klärgas- oder Deponiegasanlagen bestimmt werden kann.

Diese Aufgabe wird bei einem Verfahren der eingangs bezeichneten Art erfindungsgemäß durch die Merkmale des Patentanspruchs 1 gelöst.

Es hat sich nun überraschend herausgestellt, dass die Ionenmobilitätsspektrometrie auch unter erschwerten Einsatzbedingungen bei mit Siloxanen bzw. organischen Siliziumverbindungen verunreinigten Gasen geeignet ist und zuverlässige kontinuierliche oder diskontinuierliche Messungen des jeweiligen Siloxangehaltes ermöglicht. Bei Überschreiten von vorgegebenen Grenzwerten kann dann je nach Anlagentyp, in welchem sich das jeweilige Gas befindet, z.B. zunächst ein Warnsignal abgegeben werden oder ggf. auch die Anlage vorsorglich komplett deaktiviert werden. Bei Deponie- oder Biogas bzw. Klärgasanlagen lässt sich die Wirksamkeit der eingesetzten Filter regelmäßig überwachen und ein notwendiger Filterwechsel zum weitgehend exakt richtigen Zeitpunkt ermitteln, so dass Filterdurchbrüche zuverlässig vermieden werden. Das anthropogene und/oder biogene, methanhaltige Gas steht zumindest für die Zeit der Analyse, im kontinuierlichen Gasaustausch mit einem Ionenmobilitätsspektrometer in welchem das Gas ionisiert wird und diese Ionen anschließend in einen Driftkanal des Ionenmobilitätsspektrometers analysiert werden. Als Driftgas kann z.B. Stickstoff verwendet werden, welches in derartigen Anlagen ohne Weiteres zur Verfügung steht.

Alternativ, nicht Teil der Erfindung, kann das Verfahren auch offline durchgeführt werden, indem aus dem Gas eine Probe entnommen wird und diese Probe in ein Ionenmobilitätsspektrometer als Messgas eingeleitet wird.

Das Ionenmobilitätsspektrometer ist in den mit einem anthropogenen und/oder biogenen methanhaltigen Gas gefüllten Bereich der Biogas-, Klärgas- oder Deponiegasanlage eingebettet oder steht über eine Gasleitung mit dem Gas der Biogas-, Klärgas- oder Deponiegasanlage in Austausch. Die Gasleitung kann über ein Ventil und eine Absaugpumpe an das Ionenbeweglichkeitsspektrometer angeschlossen sein.

Wenn bei solchen Anlagen die Funktionsfähigkeit des bzw. der Gasfilter überwacht werden soll, ist bevorzugt vorgesehen, dass das Ionenmobilitätsspektrometer im Gasaustausch stromabwärts eines Gasfilters eines Gasmotors der Anlage steht.

Darüber hinaus kann das Ionenmobilitätsspektrometer oder auch ein zweites Ionenmobilitätsspektrometer im Gasaustausch stromaufwärts eines Gasfilters eines Gasmotors der Anlage stehen.

Um den Messaufwand zu reduzieren und somit schnellere und übersichtlichere Messergebnisse zu erhalten, ist bevorzugt vorgesehen, dass nur der Gehalt an einzelnen, vorausgewählten Siliziumverbindungen bestimmt wird. Typische Siloxane, die in derartigen Anlagen auftreten, sind Tetramethylsilan, Trimethylsilanol, Hexamethyldisiloxan, Hexamethylcyclotrisiloxan, Octamethylcyclotrisiloxan, Octamethylcyclotetrasiloxan, Decamethyltetrasiloxan und Decamethylcyclopentasiloxan. Um aussagekräftige Ergebnisse zu erreichen, reicht es dann z.B. aus, nur den Gehalt an Trimethylsilanol, Octamethylcyclotrisiloxan, Octamethylcyclotetrasiloxan und Decamethylcyclopentasiloxan zu bestimmen.

Die Erfindung ist nachstehend anhand der Zeichnung beispielhaft näher erläutert. Diese zeigt in
- Fig. 1: ein Ionenmobilitätsspektrometer-Chromatogramm von Biogas ohne Siloxangehalt,
- Fig. 2: ein Ionenmobilitätsspektrometer-Chromatogramm von Biogas mit Siloxangehalt und in
- Fig. 3: eine schematische Darstellung eines Messaufbaus zur Durchführung des erfindungsgemäßen Verfahrens.

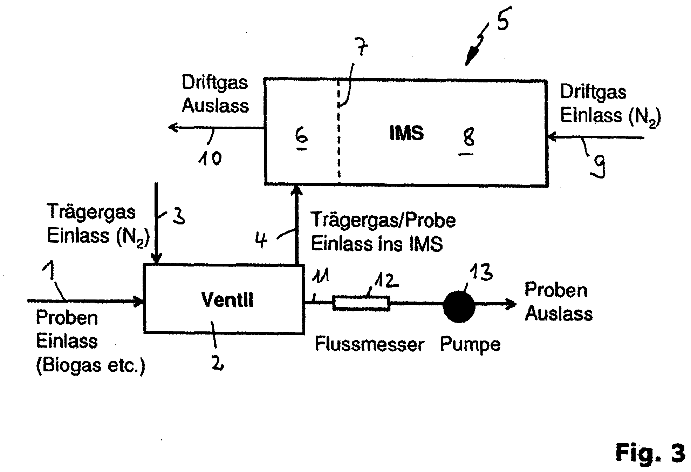

Von einer Deponie- oder Klärgasanlage ist in Fig. 3 nur eine Abzweiggasleitung 1 dargestellt, welche z.B. in direktem Kontakt mit der Anlage stromabwärts eines Gasfilters unmittelbar vor einem Gasmotor steht. Die Gasleitung 1 endet in einem Mehrwegeventil 2, welches mehrere Ein- und Ausgänge aufweist, neben der Gasleitung 1 ist als weiterer Eingang eine Trägergasleitung 3 vorgesehen. Als Auslass aus dem Ventil 2 ist eine Trägergasprobenaustrittsleitung 4 vorgesehen, die in ein allgemein mit 5 bezeichnetes Ionenmobilitätsspektrometer mündet. Dieses Ionenmobilitätsspektrometer 5 weist den üblichen Aufbau auf und ist nur schematisch angedeutet, die Leitung 4 mündet in einen Ionisationsraum 6, in welchem eine nicht dargestellte Ionisierungsquelle angeordnet ist. Der Ionisierungsraum 6 ist von einem Schaltgitter 7 zur Ionenschwarmbildung begrenzt, an welches sich ein Driftraum 8 anschließt. Am dem Gitter 7 abgewandten Ende weist der Driftraum 8 eine nicht dargestellte Sammelelektrode, beispielsweise in Form einer Faraday-Platte auf. In diesem Bereich ist auch ein Driftgaseinlass 9, z.B. für Stickstoff als Driftgas und am gegenüberliegenden Ende ein Driftgasauslass 10 vorgesehen. Das Ventil 2 weist des Weiteren eine Probenauslassleitung 11 auf, in welcher ein Flussmesser 12 und eine Pumpe 13 angeordnet sind.

Da derartige Deponiegasanlagen üblicherweise mit leichtem Unterdruck betrieben werden, ist die Pumpe 13 erforderlich, um aus dem Gassystem über die Gasleitung 1 das zu analysierende Gas dem Ionenmobilitätsspektrometer 5 zuzuführen und in diesem zu analysieren.

Wie sich herausgestellt hat, ist die Ionenmobilitätsspektrometrie ausgesprochen gut geeignet, um den Gehalt an organischen Siliziumverbindungen in Bio-, Deponie- oder Klärgasen zu bestimmen.

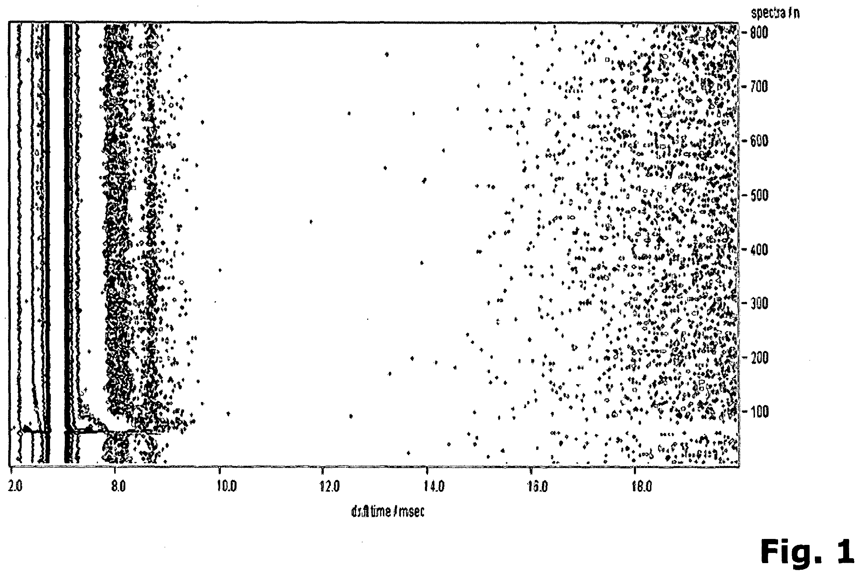

In Fig. 1 ist das Chromatogramm eines typischen Biogases ohne Siloxangehalt, d.h. eine Art Reingas dargestellt. Im linken Bereich ist lediglich der sogenannte Reaktionsionenpeak zu erkennen.

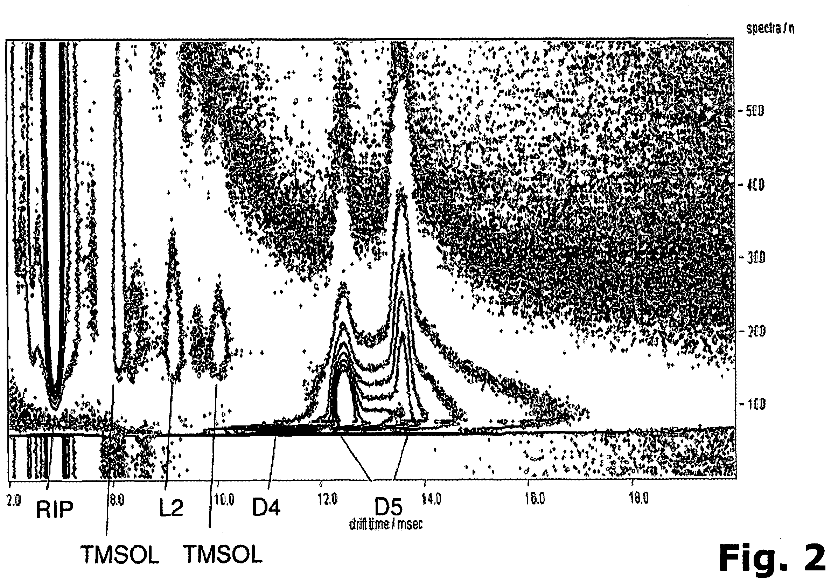

Fig. 2 zeigt dagegen ein Messergebnis eines mit Siloxanen beladenen Gases. Neben dem üblichen Reaktionsionenpeak RIP sind die Substanzen Tetramethylsilan (TMS), Hexamethyldisiloxan (L2), Octamethylcyclotetrasiloxan (D4) und Decamethylcyclopentasiloxan (D5) deutlich zu erkennen.

## Patentansprüche

1. Verfahren zur Bestimmung des Gehaltes an organischen Siliziumverbindungen in anthropogenen und/oder biogenen, methanhaltigen Klärgasen oder Deponiegasen, bei welchem die Ionenmobilitätsspektrometrie als Messverfahren verwendet wird, wobei das anthropogene und/oder biogene, methanhaltige Gas als Messgas eingesetzt wird, wobei das anthropogene und/oder biogene, methanhaltige Gas, zumindest für die Zeit der Analyse, im kontinuierlichen Gasaustausch mit einem Ionenmobilitätsspektrometer steht, in welchem das Gas ionisiert wird und diese Ionen anschließend in einen Driftkanal des Ionenmobilitätsspektrometers analysiert werden, wobei das Ionenmobilitätsspektrometer in einen mit einem anthropogenen und/ oder biogenen, methanhaltigen Gas gefüllten Bereich einer Biogas-, Klärgas- oder Deponiegasanlage eingebettet ist oder über eine Gasleitung mit dem anthropogenen und/ oder biogenen, methanhaltigen Gas der Klärgas- oder Deponiegasanlage in Austausch steht.

2. Verfahren nach Anspruch 1,
**dadurch gekennzeichnet,**
**dass** das Ionenmobilitätsspektrometer im Gasaustausch stromabwärts eines Gasfilters eines Gasmotors der Klärgas- oder Deponiegasanlage steht.

3. Verfahren nach Anspruch 1,
**dadurch gekennzeichnet,**
**dass** das Ionenmobilitätsspektrometer im Gasaustausch stromaufwärts eines Gasfilters eines Gasmotors der Klärgas- oder Deponiegasanlage steht.

4. Verfahren nach einem oder mehreren der Ansprüche 1 bis 3,
**dadurch gekennzeichnet,**
**dass** nur der Gehalt an einzelnen, vorausgewählten Siliziumverbindungen bestimmt wird.

## Claims

1. Method for determining the content of organic silicon compounds in anthropogenic and/or biogenic, methane-containing sewer gases or landfill gases, in which ion mobility spectrometry is used as a measurement method, wherein the anthropogenic and/or biogenic, methane-containing gas is used as a measurement gas, wherein the anthropogenic and/or biogenic containing gas, at least for the time of the analysis, is in continuous gas exchange with an ion-mobility spectrometer, in which the gas is ionised and said ions are subsequently analysed in a drift channel of the ion mobility spectrometer, wherein the ion mobility spectrometer is embedded in a region of a biogas-, sewage gas-, or landfill gas facility filled with an anthropogenic and/or biogenic, methane-containing gas or is in exchange via a gas line with the anthropogenic and/or biogenic, methane-containing gas of the sewage gas- or landfill gas facility.

2. Method according to claim 1,
**characterised in that**
the ion mobility spectrometer in the gas exchange is downstream of a gas filter of a gas motor of the sewage gas- or landfill gas facility.

3. Method according to claim 1,
**characterised in that**
the ion mobility spectrometer in the gas exchange is upstream of a gas filter of a gas motor of the sewage gas- or landfill gas facility.

4. Method according to one or a plurality of claims 1 to 3,
**characterised in that**
only the content of individual, pre-selected silicon compounds is determined.

## Revendications

1. Procédé pour la détermination de la teneur en composés de silicium organiques de gaz d'épuration ou de gaz de décharge anthropogènes et/ou biogènes, contenant du méthane, dans lequel on utilise comme méthode de mesure la spectrométrie de mobilité ionique, en utilisant comme gaz de mesure le gaz anthropogène et/ou biogène, contenant du méthane, le gaz anthropogène et/ou biogène, contenant du méthane, se trouvant, au moins pendant la durée de l'analyse, en échange continu de gaz avec un spectromètre de mobilité ionique, dans lequel le gaz est ionisé et ces ions sont ensuite analysés dans un canal de dérivation du spectromètre de mobilité ionique, le spectromètre de mobilité ionique étant intégré dans une zone remplie d'un gaz anthropogène et/ou biogène, contenant du méthane, d'une unité de biogaz, gaz d'épuration ou gaz de décharge ou se trouvant en échange via une conduite de gaz avec le gaz anthropogène et/ou biogène, contenant du méthane, de l'unité de gaz d'épuration ou gaz de décharge.

2. Procédé selon la revendication 1,
**caractérisé en ce que**
le spectromètre de mobilité ionique se trouve en échange de gaz en aval d'un filtre à gaz d'un moteur à gaz de l'unité de gaz d'épuration ou gaz de décharge.

3. Procédé selon la revendication 1,
**caractérisé en ce que**
le spectromètre de mobilité ionique se trouve en échange de gaz en amont d'un filtre à gaz d'un moteur à gaz de l'unité de gaz d'épuration ou gaz de décharge.

4. Procédé selon une ou plusieurs des revendications 1 à 3,
**caractérisé en ce que**
seule est déterminée la teneur en composés de silicium particuliers, choisis au préalable.
